(19) Europäisches Patentamt
European Patent Office
Office européen des brevets

(11) Publication number: 0 136 016
A2

# (12) EUROPEAN PATENT APPLICATION

(21) Application number: 84305427.1

(22) Date of filing: 09.08.84

(51) Int. Cl.⁴: C 07 D 263/04, C 07 D 263/06, A 01 N 25/32

(30) Priority: 24.08.83 US 525841

(43) Date of publication of application: 03.04.85 Bulletin 85/14

(84) Designated Contracting States: AT BE CH DE FR GB IT LI NL

(71) Applicant: STAUFFER CHEMICAL COMPANY, Westport Connecticut 06881 (US)

(72) Inventor: Gaughan, Edmund Jeremiah, 798 Wildcat Canyon Road, Berkeley California 94708 (US)
Inventor: Teach, Eugene Gordon, 1929 Downey Place, El Cerrito California 94530 (US)

(74) Representative: Smith, Sydney et al, Elkington and Fife High Holborn House 52/54 High Holborn, London WC1V 6SH (GB)

## (54) N-(Substituted)-5-(substituted phenyl)-1,3-Oxazolidines: Herbicidal antidotes.

(57) N-(haloacyl)-5-(substituted phenyl)-oxazolidines as new compounds having the formula

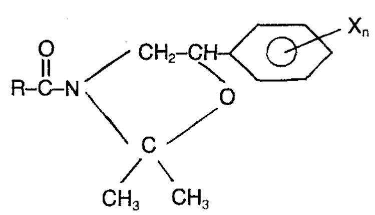

wherein R is $C_1$–$C_{10}$ haloalkyl, $C_1$–$C_{20}$ alkyl, $C_1$–$C_6$ cycloalkyl, $C_1$–$C_6$ haloalkenyl or $C_1$–$C_6$ alkylthio; X is $C_1$–$C_4$ alkyl, $C_1$–$C_4$ alkoxy, $C_1$–$C_4$ haloalkyl or halogen; and n is the integer 1 to 5, inclusive, with the proviso that where X is trifluoromethyl, R is other than 3-chloropropyl; useful as antidotes to protect against thiocarbamate or acetanilide herbicides when applied by various methods and improved herbicidal composition comprising an herbicidally effective amount of a thiocarbamate or acetanilide herbicide and an antidotally effective amount of said antidote compounds.

EP 0 136 016 A2

# N-(SUBSTITUTED)-5-(SUBSTITUTED PHENYL)-1,3-OXAZOLIDINES: HERBICIDAL ANTIDOTES

## Field of the Invention

This invention relates to a novel class of N-(substituted) oxazolidines useful as active herbicidal antidotes to protect against and decrease crop injury caused by thiocarbamate and acetanilide herbicides. The oxazolidine ring of these antidotes is substituted at the 5-position with a phenyl group which is in turn substituted with one or more alkyl, alkoxy, haloalkyl groups or halogens. Other classes of haloacyl oxazolidine herbicide antidotes are known, but none with substituted phenyl groups at the 5-position of the oxazolidine ring.

The following U.S. Patents disclose oxazolidine herbicide antidotes, however, none of such antidotes contain either a substituted or unsubstituted phenyl group at the 5-position of the oxazolidine ring: U.S. Patent Nos. 3,959,304; 3,989,503; 4,124,372; 4,186,130; 4,236,011; 4,243,811; 4,259,500; 4,268,677; 4,269,986; 4,299,964; 4,319,031; 4,350,517; and 4,354,867. U.S. Patent Nos. 4,072,688 and 4,322,240 disclose haloacyl oxazolidine herbicidal antidotes wherein an unsubstituted phenyl group is a possible substituent at the 5-position of the oxazolidine ring, and a substituted phenyl is a possible substituent at the 2-position. U.S. Patent No. 4,249,931 discloses, among other oxazolidine antidotes, haloacyl oxazolidine antidotes wherein p-chlorophenyl or p-bromophenyl are possible substituents at the 2-position of the oxazolidine ring, whereas an alkoxy- or alkylthio- methyl group is a possible substituent at the 5-position. U.S. Patent 4,249,932 discloses, among other oxazolidine antidotes, 5-substituted 3-haloacyl oxazolidines, wherein phenoxymethyl is a substituent at the 5-position of the oxazolidine ring.

Thus, none of the above patents disclose haloacyl oxazolidine antidotes wherein there is a substituted phenyl group at the 5-position.

## Background of the Invention

An herbicide is a compound which adversely controls or modifies plant growth, e.g., killing, retarding, defoliating, desiccating, regulating, stunting, tillering, stimulating, and dwarfing. The term "plant" refers to all physical parts of a plant, including seeds, seedlings, saplings, roots, tubers, stems, stalks, foliage, and fruits. "Plant growth" includes all phases of development from seed germination to natural or induced cessation of life.

Herbicides are generally used to control or eradicate weed pests. They have gained a high degree of commercial success because it has been shown that such control can increase crop yield and reduce harvesting costs. Thiocarbamate and acetanilide herbicides are particularly effective in the control of grassy type weeds which interfere with the cultivation of a wide variety of crops, e.g., barley, corn, cotton, lentils, peanuts, peas, potatoes, soybeans, spinach, tobacco and tomatoes. Frequently the effective use of these herbicides requires the addition of an antidote compound to increase the tolerance of these compounds to the herbicides.

The most popular methods of herbicide application include: pre plant incorporation into the soil; in-furrow application to seeds and surrounding soil; pre-emergence surface treatment of seeded soil; and post-emergence treatment of the plant and soil.

A manufacturer of an herbicide generally recommends a range of application rates and concentrations calculated to maximize weed control. The range of rates varies from approximately 0.01 to 50 pounds per acre (0.0112 to 56 kilograms per hectare (k/ha)), and is usually in the range of from 0.1 to 25 pounds per acre (0.112 to 28 k/ha). The term "herbicidally effective amount" describes the amount of an herbicide compound which controls or modifies plant growth. The actual amount used depends upon several considerations, including particular weed susceptibility and over all cost limitations.

While many herbicides are immediately toxic to a large number of weed pests, it is known that the effect of many herbicides upon important plant cultivations is either non-selective or not adequately selective.

Thus, many herbicides damage not only the weeds to be controlled, but to a greater or lesser extent, the desirable cultivated plants as well. This holds true for many herbicidal compounds which have been commercially successful and are commercially available. These herbicides include types such as triazines, urea derivatives, halogenated acetanilides; carbamates, thiocarbamates, thiocarbamate sulfoxides, pyrrolidinones, and the like. Some examples of these compounds are described in U.S. Patent Nos. 2,891,855, 2,913,237, 3,037,853, 3,175,897, 3,185,720, 3,198,786, 3,442,945, 3,582,314, 3,780,090, 3,952,056 4,110,105 and 3,257,190.

The side effect of injury to a cultivated crop by various herbicides is particularly inconvenient and unfortunate. Acetanilide and thiocarbamate herbicides can sometimes cause malformation and stunting of crop plants. This abnormal growth in the crop plant results in loss of crop yield.

Previous attempts are described to overcome this problem. The treatment of the crop seed with certain "hormonal" antagonistic agents to planting is described, see U.S. Patents 3,131,509 and 3,564,768. The protective agents, as well as the herbicide, in these prior processes are largely specific to certain cultivated plant species or in the nature of the antagonistic agents. The prior antagonistic agents have not been notably successful. The aforementioned patents specifically exemplify and describe the treatment of seeds employing compounds of a different chemical class not suggestive of the present invention. Other herbicidal antidotes are disclosed in U.S. Patent Nos. 4,021,224 and 4,021,229.

The precise mechanism by which an antidote reduces herbicidal crop injury has not been established. An antidote compound may be a remedy, interferent, protectant, or antagonist. As used herein, "antidote" describes a compound which has the effect of establishing herbicide selectivity, i.e., continued herbicidal phytotoxicity to weed species and reduced or non-phytotoxicity to cultivated crop species. The term "antidotally effective amount" describes the amount of an antidote compound which counteracts a phytotoxic response of a beneficial crop to an herbicide.

Description of the Invention

It has now been discovered that N-substituted-5-(substituted phenyl)-1,3-oxazolidines are effective antidotes for the protection of a variety of crops from thiocarbamate and acetanilide herbicide injuries. Such antidotal effects as decreasing crop injury and increasing crop tolerance to such herbicides are manifested when such herbicides and antidotes are applied in a variety of ways, for example, independently, or together in tank mixtures or in combination with other compounds.

The N-substituted-5-(substituted phenyl)-1,3- oxazolidines disclosed herein are considered novel and correspond to the following formula:

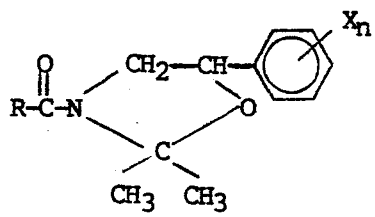

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

Preferred antidotes of this novel class of compounds are the N-haloacyl-5-(substituted phenyl)-1,3-oxazolidines which have acyl groups wherein R is $C_1$-$C_4$ haloalkyl, $C_1$-$C_4$ alkyl, $C_1$-$C_4$ cycloalkyl, $C_1$-$C_4$ haloalkenyl and $C_1$-$C_4$ alkylthio; and phenyl substituents wherein X is $C_1$-$C_2$ alkyl, $C_1$-$C_2$ alkoxy, $C_1$-$C_2$ haloalkyl, chlorine, bromine or fluorine; and n is 1, 2 or 3.

It is further preferred that R be methyl or ethyl groups which are mono-, di- or tri-substituted with halogen; that X be methyl, methoxy, halogen or trifluoromethyl; and that n = 1, 2 or 3.

It is even more preferred that R be mono- or di-chloromethyl; X be para-chloro, para-methyl, meta- or para-methoxy, or meta-trifluoromethyl; and n = 1 or 3.

It is most preferred that R be dichloromethyl; that X be para-methoxy, or meta-trifluoromethyl; and n = 1.

This invention also embodies a two-part herbicidal system comprising

(a) an herbicidally effective amount of a thiocarbamate compound of the formula

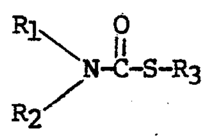

in which

$R_1$ is alkyl having 1-6 carbon atoms, inclusive;

$R_2$ is selected from the group consisting of alkyl having 1-6 carbon atoms, inclusive; and cyclohexyl; or

$R_1$ and $R_2$ form indistinguishable parts of a single alkylene ring having 4-10 carbon atoms, inclusive; and

$R_3$ is selected from the group consisting of alkyl having 1-6 carbon atoms, inclusive; haloalkyl wherein halo is selected from the group consisting of chlorine, bromine and iodine; alkenyl having 2-6 carbon atoms, inclusive; haloalkenyl wherein halo is selected from the group consisting of chlorine, bromine and iodine and alkenyl having 2-6 carbon atoms, inclusive; benzyl and halo-substituted benzyl, wherein halo is selected from the group consisting of chlorine, bromine and iodine; and

(b) a non-phytotoxic antidotally effective amount of a compound of the formula

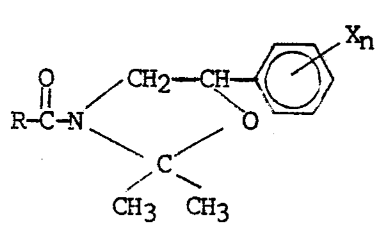

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

The preferred thiocarbamates of such compositions are those wherein $R_1$ and $R_3$ are $C_1$-$C_6$ alkyl groups and $R_2$ is either $C_1$-$C_6$ alkyl or $C_1$-$C_8$ cycloalkyl. The most preferred thiocarbamates are S-propyl N,N-dipropyl thiocarbamate; S-ethyl N,N-dipropyl thiocarbamate; S-ethyl N,N-diisobutyl thiocarbamate; and S-ethyl-N-ethyl-N-cyclohexyl thiocarbamate.

The preferred and most preferred antidotes of the compositions including thiocarbamates are delineated above in the discussion of the novelty of the instant compounds.

The invention also embodies a two-part herbicidal system comprising

(a) an herbicidally effective amount of an acetanilide compound of the formula

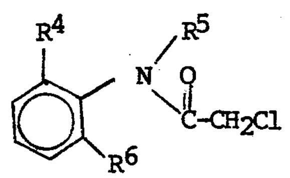

in which

$R^4$ and $R^6$ are independently selected from the group consisting of hydrogen and alkyl having 1-6 carbon atoms, inclusive; and

$R^5$ is selected from the group consisting of alkyl having 1-6 carbon atoms, inclusive; alkoxyalkyl having 1-8 carbon atoms, inclusive; and carbethoxyalkyl wherein the alkyl group has 1-4 carbon atoms, inclusive; and

(b) a non-phytotoxic antidotally effective amount of a compound of the formula

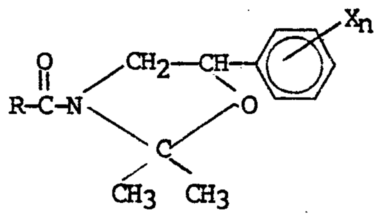

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

The preferred acetanilides of such composition are those wherein $R_4$ and $R_6$ are each independently alkyl having 1-4, or more preferably 1-2, carbon atoms, inclusive; and $R_5$ is alkoxyalkyl having 1-4 carbon atoms, inclusive.

By way of exemplification, the active acetanilide compounds employed in the invention may include: 2-chloro-2',6'-diethyl-N-(methoxymethyl) acetanilide; 2-chloro-2'-methyl-6'-ethyl-N-(2-methoxypropyl)-acetanilide; 2-chloro-2',6'-dimethyl-N-(methoxyethyl) acetanilide; 2-chloro-2'-methyl-6'-ethyl-N-(ethoxymethyl) acetanilide; 2-chloro-N-isopropyl acetanilide; 2-chloro-2',6'-diethyl-N-(n-butoxymethyl) acetanilide; 2-chloro-N-carbethoxymethyl-2',6'-diethyl acetanilide; and 2-chloro-2'-methyl-6'-ethyl-N-(2-methoxy-1-methylethyl) acetanilide.

The most preferred acetanilides of such compositions are 2-chloro-2',6'-diethyl-N-(methoxymethyl) acetanilide and 2-chloro-2',6'dimethyl-N-(methoxymethyl) acetanilide.

The most preferred antidote of such acetanilide/antidote composition is 2,2-dimethyl-3-(dichloroacetyl)-5-(p-methoxyphenyl)-1,3-oxazolidine.

This invention also relates to a method of controlling undesirable vegetation and reducing crop injury caused by thiocarbamate and

acetanilide herbicides which comprises applying to the locus where control is desired a compound of the formula

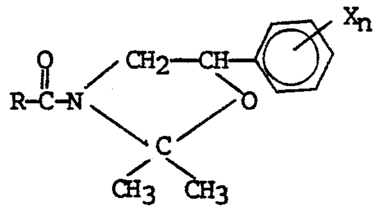

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_2$-$C_4$ haloalkyl or halogen; and

n is the integer 1 to 5, inclusive,

with the proviso that where X is trifluoromethyl, R cannot be 3-chloropropyl.

The locus where control and herbicidal crop injury reduction is desired may include soil, seeds, seedlings and vegetation.

## Preparation

In general, the active antidote compounds of the present invention can be prepared by the following methods.

An appropriate benzaldehyde is reacted with trimethylsilyl cyanide (TMSCN) to produce an adduct. The adduct is then reduced to a substituted phenyl amino alcohol.

The oxazolidine intermediates are prepared by condensing the amino alcohol with dimethyl ketone in boiling toluene with the continuous separation of water. This method is essentially described by Bergmann et al., J.A.C.S., 75:385 (1953). Aliquots of these solutions are then used to prepare the copounds of this invention.

The oxazolidine intermediate is then reacted with the appropriate acid halide in the presence of a hydrogen halide acceptor, such as triethylamine, to produce the desired N-haloacyl-5-(substituted phenyl) oxazolidine. Work-up and purification procedures involved standard methods of extraction, distillation or crystallization.

U.S. Patent Nos. 4,038,284 and 4,219,655 describe processes for the production of other N-acyl substituted oxazolidines.

The compounds of the present invention and their preparation are more particularly illustrated by the following examples. Following the examples of preparation is a table of compounds which are prepared according to the procedures described herein. Compound numbers have been assigned to them and are used for identification throughout the balance of the specification.

## EXAMPLE I

### Preparation of 2,2-Dimethyl-3-(dichloroacetyl)-5-(p-methoxyphenyl)-1,3-oxazolidine

1. Preparation of Adduct of p-Methoxybenzaldehyde and Trimethylsilyl cyanide (TMSCN)

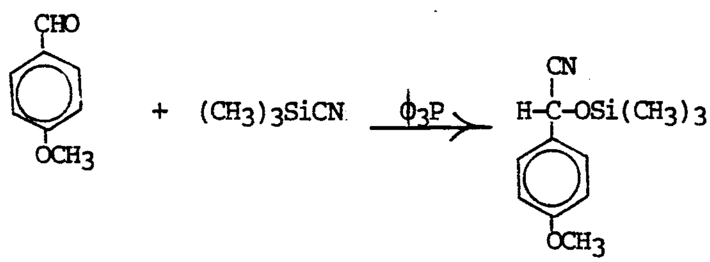


Ten and nine-tenths grams (10.9 g) (0.11 mole) of TMSCN containing about 5 milligrams (mg) of triphenylphosphine was added dropwise with stirring under an argon atmosphere to 13.6 g (0.10 mole) of p-methoxybenzaldehyde. The temperature rose by 4-5°C.

The mixture was stirred for 45 minutes, and then a sample was examined by gas chromatography (g.c.) which indicated about 11% product. The mixture was then stirred overnight at ambient temperature. In the morning g.c. analysis indicated 64% product and 33% unreacted aldehyde.

The mixture was warmed to about 80°C for two hours. Gas chromatography showed 78% product and 18% aldehyde. One milliliter (ml) of TMSCN and a trace of triphenylphosphine were added, and the mixture was then warmed to 70-80°C for two hours. Gas chromatographic analysis indicated 83% product.

The reaction mixture was then allowed to stand for two days at room temperature. An additional 3 ml of TMSCN and a trace of triphenylphosphine was then added to the mixture. For two hours, the mixture was heated to maintain a temperature range of 70-80°C. Two ml of TMSCN was added, and the temperature was raised to 130-140°C and maintained for another 2.5 hours. The g.c. area percent was 99%. The reaction mixture was evaporated _in vacuo_. The product was a red-yellow liquid. The yield was 22.7 g (96.6%). The IR and NMR spectra were consistent with the expected structure.

2. Preparation of 1-(p-Methoxyphenyl)-2-aminoethanol

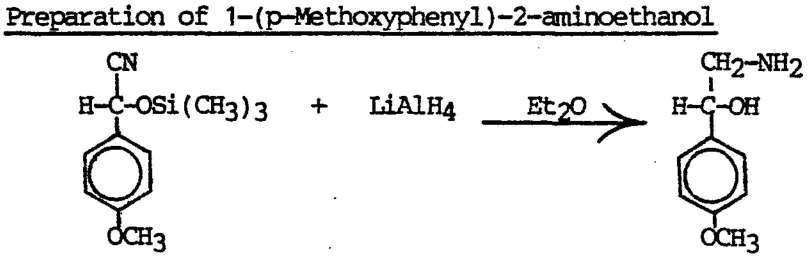


Thirteen grams (0.055 mole) of the product of Step 1, the above cyanohydrin trimethylsilyl ether, in 30 ml of dry diethylether was added dropwise to a suspension of 2.5 g (0.067 mole) of lithium aluminum hydride in 120 ml of the same solvent. The mixture refluxed spontaneously during the addition. The mixture was then stirred for 1.5 hours at room temperature.

Two and forty-three hundredths ml (2.43) of water, 2.43 ml of a 15% aqueous sodium hydroxide solution and then 7.35 ml of water were carefully added dropwise to the reaction mixture, which was diluted with additional ether during the additions.

The mixture was filtered; the filter-cake was washed with ether and methylene chloride. The combined filtrate and washings were washed with brine; the aqueous layer was then extracted with ether. The organic phase was dried over $Na_2SO_4$; the solvents were removed _in vacuo_.

The structure of the expected product was confirmed by an NMR spectrum. It was a waxy solid with a g.c. area percent of 94.5%. The yield was 5.3 g (57.6%).

3. <u>Preparation of 2,2-Dimethyl-3-(dichloroacetyl)-5-(p-methoxyphenyl)-1,3-oxadolidine</u>

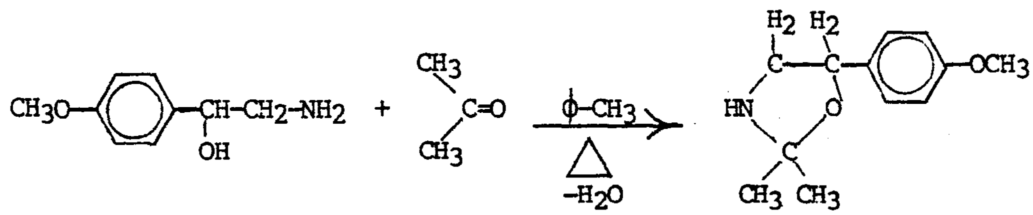


Two and five-tenths g (2.5) of acetone was added to 4.8 g (0.029 mole) of the amino alcohol product of Step 2 in 40 ml of toluene. The mixture was refluxed at 200-400 mm and at approximately 80°C under a Dean-Stark water take-off tube and reflux condenser until about 0.4 ml of water was collected. The solution was dried over $Na_2SO_4$ to a final volume of 57 ml.

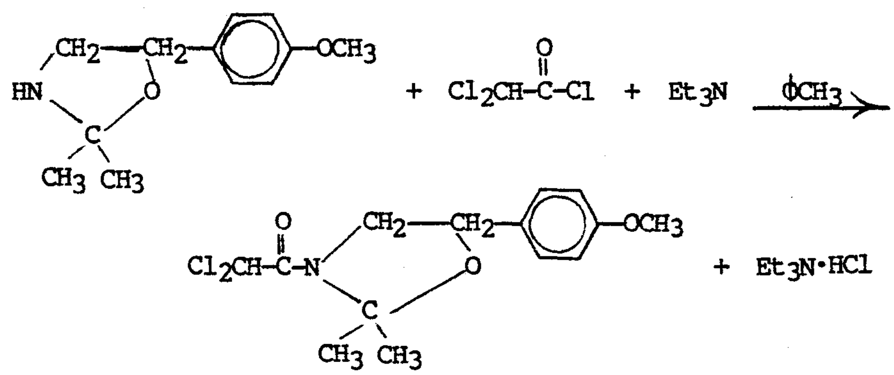


To 42.4 ml of a stock solution of the above oxazolidine product was added 2.1 g of triethylamine. Two and nine-tenths g of dichloroacetyl chloride in 8 ml of toluene was added dropwise to the oxazolidine solution at about 0°C. The mixture was stirred for three hours and filtered. The filtrate was washed with water, sodium bicarbonate solution, brine and dried over $MgSO_4$. The solvent was removed <u>in vacuo</u>. The product was a tan semi-solid. The yield was 5.0 g (79.4%). The structure was confirmed by IR and NMR spectra.

EXAMPLE II

Preparation of 2,2-Dimethyl-3-(dichloroacetyl)-5-(p-chlorophenyl)-1,3-oxazolidine

Ten ml of toluene and 2.0 g of triethylamine was added to 16.8 ml of the appropriate oxazolidine stock solution (4.2 g; 0.02 mole) in toluene prepared according to Steps 1-3 of Example I. A solution of 2.7 g of dichloroacetyl chloride in 10 ml of toluene was added to the mixture at a temperature ranging from 0-5°C. The mixture was worked up as described in Step 4 of Example I.

The product had a melting point of 95-100°C. The yield was 4.2 g (65.1%). The structure was confirmed by IR and NMR spectra.

EXAMPLE III

Preparation of 2,2-Dimethyl-3-(chloroacetyl)-5-(p-methylphenyl)-1,3-oxazolidine

To 12 ml of a toluene stock solution containing 0.0157 mole of 2,2-dimethyl-5-(p-methylphenyl)-1,3-oxazolidine (prepared in accordance with Steps 1-3 of Example I) was added an additional 75 ml of toluene. At -5°C, 1.25 ml (0.0157 mole) of chloroacetyl chloride was added, followed by the addition of 1.25 ml (0.0157 mole) of triethylamine at the same temperature. The mixture was stirred, and its temperature was allowed to become ambient. The mixture was washed with a sodium bicarbonate solution, water, and then dried over $Na_2SO_4$. The solvent was removed *in vacuo*. The product was an oil, $n_D^{30}$ = 1.5382. The yield was 3.7 g (88%).

The structure was confirmed by IR spectrum.

EXAMPLE IV

Preparation of 2,2-Dimethyl-3-(trichloroacetyl)-5-(p-methylphenyl)-1,3-oxazolidine

The appropriate oxazolidine was prepared according to Example I. and the reaction procedure was carried out as therein detailed and on the same scale. Trichloroacetyl chloride (1.75 ml, 0.0157 mole) was the acylating agent.

The product was a tan solid with a melting point between 105-107°C. The yield was 4.4 g (83.3%). The structure was confirmed by IR spectrum.

TABLE I

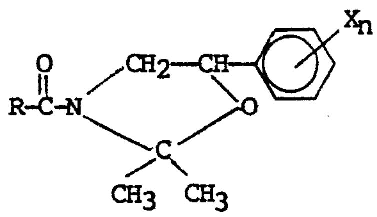

| Compound Number | $X_n$ | R | n |
|---|---|---|---|
| 1 | 4-methoxy | $-CHCl_2$ | 1 |
| 2 | 2,4-dichloro | $-CHCl_2$ | 2 |
| 3 | 4-chloro | $-CHCl_2$ | 1 |
| 4 | 3-trifluoromethyl | $-CHCl_2$ | 1 |
| 5 | 4-methyl | $-CH_2Cl$ | 1 |
| 6 | 4-methyl | $-CHCl_2$ | 1 |
| 7 | 4-methyl | $-CCl_3$ | 1 |
| 8 | 4-methyl | $-(CH_2)_3Cl$ | 1 |
| 9 | 2,4-dimethyl | $-CHCl_2$ | 2 |
| 10 | 4-chloro | $-(CH_2)_2Br$ | 1 |
| 11 | 4-chloro | $-CH_2Cl$ | 1 |
| 12 | 3-trifluoromethyl | $-CH_2Cl$ | 1 |
| 13 | 3-trifluoromethyl | $-C(Br)=CH_2$ | 1 |
| 14 | 3-trifluoromethyl | 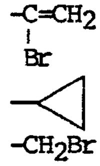 | 1 |
| 15 | 3-trifluoromethyl | $-CH_2Br$ | 1 |
| 16 | 3-trifluoromethyl | $-(CH_2)_{14}CH_3$ | |
| 17 | 3-trifluoromethyl | $-SC_3H_7$ | 1 |
| 18 | 3,4,5-trimethoxy | $-CH_2Br$ | 3 |
| 19 | 3,4,5-trimethoxy | $-CHCl_2$ | 3 |
| 20 | 3,4,5-trimethoxy | $-C(Cl)=CCl_2$ | 3 |

Testing

Stock solutions of the herbicides were prepared by diluting the requisite amount of each herbicide in water or in a 1:1 mixture of water and acetone. Examples of solution compositions and application rates are summarized in Table II.

TABLE II

Herbicide Stock Solutions

| Herbicide Name | Composition: Herbicide (mg)* | Composition: Water (ml) | Acetone (ml) | Application: ml/flat** | Application: lb/acre |
|---|---|---|---|---|---|
| VERNAM® 6.7LE S-propyl N,N-dipropyl thiocarbamate | 546 | 500 | 0 | 4 | 1.00 |
| | 3276 | 500 | 0 | 4 | 6.00 |
| LASSO® 4E 2-chloro-2',6'-diethyl-N-(methoxymethyl) acetanilide | 7656 | 350 | 350 | L.S.T.*** | 3.50 |

The VERNAM® 6.7LE formulation contains about 80% active herbicide compound. The LASSO® 4E formulation contains about 48% active herbicide compound.

* The weight is measured in terms of mg of formulated herbicide.

** The flats measure 5.95 inches by 9.5 inches. Approximately four (4) mg/flat is equal to one (1) lb/acre.

*** Solution applied using a linear spray table at a rate of 80 gallons/-acre.

The herbicide was either incorporated into the soil prior to planting or applied to the soil after planting and prior to emergence of the plants. In some cases of pre-plant incorporation, the herbicide was incorporated into the soil alone in preparation for in-furrow application of the antidote; in others the herbicide solution was tankmixed with the antidote solution prior to incorporation.

These methods are abbreviated in the tables as follows:

PPI = pre-plant incorporation;

PPI-TM = pre-plant incorporation in tank-mix with the antidote; and

PES = pre-emergence surface.

Stock solutions of each antidote compound were prepared at the desired concentrations by diluting the requisite amounts of each antidote in acetone.

The antidote solutions were applied to the soil either by in-furrow surface application or by pre-plant incorporation. In all cases of pre-plant incorporation, the antidote was tank-mixed with the herbicide prior to incorporation into the soil. These methods are abbreviated in the tables as follows:

IF = in-furrow surface application of antidote; and

PPI-TM = pre-plant incorporation of antidote, in tank-mix with herbicide.

For in-furrow application, a one pint (473 cubic centimeter (cc)) sample of soil containing the previously incorporated herbicide was removed and retained from each planting flat. After leveling and furrowing the soil, seeds of the crop or weed species were planted 1/2 inch deep (1.27 centimeter). Each flat was divided in half by a wooden barrier. A stock solution of the antidote was atomized directly onto the exposed seeds and soil in the open furrow on one side of the barrier. The seeds in the entire flat were then covered with the previously removed soil. The antidotally untreated sections of flats were compared for observed differences which would indicate lateral movement of the antidote through the soil.

Control flats contained crops treated with herbicide only.

All flats were placed on greenhouse benches where temperature was maintained between 70 and 90°F (21.1 to 32.2°C). The flats were watered by sprinkling as needed to assure good plant growth.

All of the soil used in the tests described herein was loamy sand soil treated with 50 parts per million (ppm) each of a commercially available fungicide, N-[(trichloromethyl)-thio]-4-cyclohexene-1,2-dicarboximide, and 17-17-17 fertilizer, which contains 17% by weight equivalent each of nitrogen, phosphorus pentoxide, and potassium oxide.

Injury ratings were taken four weeks after application of the antidote. The effectiveness of the antidote was determined by visual comparison of crop injury which occurred in the test flats to that which occurred in the control flats.

The treated crops initially screened for diminution of herbicidal injury were milo, wheat (WHT), cotton (COT), rice, barley (BAR), corn and soybeans (SOY). The compound was also tested on the following weeds: watergrass (WG) (Echinochloa crusgalli); foxtail (FT) (Stearia viridis); wild oats (WO) (Avena fatua); and shattercane (SC) (Sorghum bicolor).

The following are the herbicides with which the antidotes were tested:

| | | |
|---|---|---|
| VERNAM® | - | S-propyl N,N-dipropyl thiocarbamate |
| EPTC® | - | S-ethyl-N,N-dipropyl thiocarbamate |
| RO-NEET® | - | S-ethyl-N-ethyl-N-cyclohexyl thiocarbamate |
| SUTAN® | - | S-ethyl-N,N-diisopropyl thiocarbamate |
| LASSO® | - | 2-chloro-2',6'-diethyl-N-(methoxymethyl) acetanilide |
| TERIDOX® | - | 2-chloro-5',6'-dimethyl-N-(methoxyethyl) acetanilide |

The results are indicated in Tables III and IV by numbers separated by slashes, for eg., *10/65. The first number is the percentage of injuries sustained by the indicated crop or weed species when treated by both the antidote and herbicide at the rates specified. The second number is the percentage of injuries sustained by the crop or weed species when treated by the herbicide alone at the rate specified. Thus, the numbers represent: Antidote treated/Antidote untreated.

An asterisk (*) before the rating numbers in Table III indicates that the antidote is active in reducing herbicidal injury to the crop. A double asterisk (**) after the number indicates that the rating was an average of two trials.

All rates shown, for both herbicide and antidote, are in pounds per acre.

Injury Ratings

The injury to the crop (Table III) or weeds (Table IV) is shown as a percentage of damage done to the plants as compared to an evaluation of the overall undamaged state of the plants indicated by check flats not treated with either herbicide or antidote. The damage done to the plants is a function of the number of plants injured and the extent of injury to each plant. This rating is made four (4) weeks after application of the herbicide alone or of the herbicide in combination with the antidote.

Table IV shows that the antidote compound has no effect on weeds, i.e., herbicidal injury to the weeds is sustained even in the presence of an antidote compound.

TABLE III

Antidotal Effectiveness

| Herbicide | | | Antidote | | | % Injury | | | | | | |
|---|---|---|---|---|---|---|---|---|---|---|---|---|
| Name | Rate lb/A | Method | Cmpd. No. | Rate lb/A | Method | Milo | Wheat | Cotton | Rice | Barley | Corn | Soybean |
| VERNAM | 1.00 | PPI | 1 | 5.00 | IF | *15/90 | 90/90 | *15/35 | 90/90 | 55/55 | | |
| VERNAM | 6.00 | PPI | 1 | 5.00 | IF | | | | | | *10/90 | 75/40 |
| RONEET | 3.00 | PPI | 1 | 5.00 | IF | *25/70 | | | | | | |
| RONEET | 3.00 | PPI | 1 | 5.00 | IF | *15/70 | | | | | | |
| RONEET | 3.00 | PPI/TM | 1 | 1.00 | PPI/TM | *15/75 | | | | | | |
| RONEET | 3.00 | PPI/TM | 1 | 2.00 | PPI/TM | *20/75 | | | | | | |
| RONEET | 3.00 | PPI/TM | 1 | 5.00 | PPI/TM | * 0/75 | | | | | | |
| RONEET | 3.00 | PPI/TM | 1 | 5.00 | PPI/TM | * 0/65 | | | | | | |
| RONEET | 3.00 | PPI/TM | 1 | 1.00 | PPI/TM | * 0/65 | | | | | | |
| RONEET | 3.00 | PPI/TM | 1 | 0.50 | PPI/TM | *15/65 | | | | | | |
| RONEET | 3.00 | PPI/TM | 1 | 0.25 | PPI/TM | *30/65 | | | | | | |
| EPTC | 6.00 | PPI/TM | 1 | 0.05 | PPI/TM | | | | | | *30/85** | |
| EPTC | 6.00 | PPI/TM | 1 | 0.50 | PPI/TM | | | | | | * 0/75 | |
| EPTC | 6.00 | PPI/TM | 1 | 5.00 | PPI/TM | | | | | | *10/75 | |
| EPTC | 6.00 | PPI/TM | 1 | 0.025 | PPI/TM | | | | | | 80/90 | |
| EPTC | 6.00 | PPI/TM | 1 | 0.012 | PPI/TM | | | | | | 85/90 | |
| LASSO | 3.50 | PES | 1 | 5.00 | IF | *30/95 | 70/70 | | 100/100 | 70/70 | | |
| TERIDOX | 1.00 | PES | 1 | 5.00 | IF | *80/100 | 70/70 | | | 80/80 | *40/100 | |
| SUTAN | 6.00 | PPI | 1 | 1.00 | IF | | | 70/70 | | | | |
| SUTAN | 6.00 | PPI | 1 | 5.00 | IF | | | 70/70 | | | | |
| VERNAM | 6.00 | PPI/TM | 1 | 1.00 | PPI/TM | | | | | | | 60/60 |
| VERNAM | 6.00 | PPI/TM | 1 | 2.00 | PPI/TM | | | | | | | 85/60 |
| VERNAM | 6.00 | PPI/TM | 1 | 5.00 | PPI/TM | | | | | | | 85/60 |
| VERNAM | 5.00 | PPI | 1 | 1.00 | IF | | | 40/40 | | | | |
| VERNAM | 5.00 | PPI | 1 | 5.00 | IF | | | 40/40 | | | | |

TABLE III
(continued)

| Herbicide | | | Antidote | | | % Injury | | | | | | |
|---|---|---|---|---|---|---|---|---|---|---|---|---|
| Name | Rate lb/A | Method | Cmpd. No. | Rate lb/A | Method | Milo | Wheat | Cotton | Rice | Barley | Corn | Soybean |
| VERNAM | 1.25 | PPI | 3 | 5.00 | IF | *35/99 | *75/99 | 40/40 | *75/95 | 85/85 | | |
| VERNAM | 6.00 | PPI | 3 | 5.00 | IF | | | | | | *75/90 | 35/40 |
| RONEET | 3.00 | PPI | 3 | 5.00 | IF | *25/80 | | | | | | |
| RONEET | 3.00 | PPI | 3 | 1.00 | IF | *50/80 | | | | | | |
| RONEET | 3.00 | PPI/TM | 3 | 1.00 | PPI/TM | *60/80 | | | | | | |
| RONEET | 3.00 | PPI/TM | 3 | 2.00 | PPI/TM | *20/80 | | | | | | |
| RONEET | 3.00 | PPI/TM | 3 | 5.00 | PPI/TM | *30/80 | | | | | | |
| VERNAM | 1.25 | PPI | 4 | 5.00 | IF | *40/100 | *80/95 | 65/65 | 85/98 | 85/85 | | |
| VERNAM | 6.00 | PPI | 4 | 5.00 | IF | | | | | | *40/95 | 90/65 |
| RONEET | 3.00 | PPI | 4 | 1.00 | IF | *20/70 | | | | | | |
| RONEET | 3.00 | PPI | 4 | 5.00 | IF | *15/70 | | | | | | |
| EPTC | 6.00 | PPI/TM | 4 | 0.05 | PPI/TM | | | | | | *53/80** | |
| EPTC | 6.00 | PPI/TM | 4 | 0.50 | PPI/TM | | | | | | *10/70 | |
| EPTC | 6.00 | PPI/TM | 4 | 5.00 | PPI/TM | | | | | | *15/70 | |
| EPTC | 6.00 | PPI/TM | 4 | 0.025 | PPI/TM | | | | | | 85/90 | |
| EPTC | 6.00 | PPI/TM | 4 | 0.012 | PPI/TM | | | | | | 90/90 | |
| RONEET | 3.00 | PPI/TM | 4 | 1.00 | PPI/TM | *10/75 | | | | | | |
| RONEET | 3.00 | PPI/TM | 4 | 2.00 | PPI/TM | *30/75 | | | | | | |
| RONEET | 3.00 | PPI/TM | 4 | 5.00 | PPI/TM | *25/75 | | | | | | |
| RONEET | 3.00 | PPI | 4 | 1.00 | IF | *40/90 | | | | | | |
| RONEET | 3.00 | PPI | 4 | 5.00 | IF | *30/90 | | | | | | |
| RONEET | 3.00 | PPI/TM | 4 | 5.00 | PPI/TM | * 0/65 | | | | | | |
| RONEET | 3.00 | PPI/TM | 4 | 1.00 | PPI/TM | * 0/65 | | | | | | |
| RONEET | 3.00 | PPI/TM | 4 | 0.50 | PPI/TM | *30/68** | | | | | | |
| RONEET | 3.00 | PPI/TM | 4 | 0.25 | PPI/TM | *28/68** | | | | | | |

TABLE III
(continued)

| Herbicide | | | Antidote | | | % Injury | | | | | | |
|---|---|---|---|---|---|---|---|---|---|---|---|---|
| Name | Rate lb/A | Method | Cmpd. No. | Rate lb/A | Method | Milo | Wheat | Cotton | Rice | Barley | Corn | Soybean |
| RONEET | 3.00 | PPI/TM | 4 | 2.00 | PPI/TM | *30/70 | | | | | | |
| RONEET | 3.00 | PPI/TM | 4 | 2.00 | PPI/TM | *35/70 | | | | | | |
| RONEET | 3.00 | PPI/TM | 4 | 0.125 | PPI/TM | *55/70 | | | | | | |
| RONEET | 3.00 | PPI/TM | 4 | 0.06 | PPI/TM | 65/70 | | | | | | |
| VERNAM | 5.00 | PPI/TM | 4 | 1.00 | PPI/TM | | | | | | | 40/40 |
| VERNAM | 5.00 | PPI/TM | 4 | 2.00 | PPI/TM | | | | | | | 55/40 |
| VERNAM | 5.00 | PPI/TM | 4 | 5.00 | PPI/TM | | | | | | | 55/40 |
| VERNAM | 1.25 | PPI | 5 | 5.00 | IF | *75/100 | 85/95 | 60/60 | *85/97 | 90/90 | | |
| VERNAM | 6.00 | PPI | 5 | 5.00 | IF | | | | | | *20/95 | 75/65 |
| EPTC | 6.00 | PPI/TM | 5 | 0.05 | PPI/TM | | | | | | *55/80** | |
| EPTC | 6.00 | PPI/TM | 5 | 0.50 | PPI/TM | | | | | | *10/70 | |
| EPTC | 6.00 | PPI/TM | 5 | 5.00 | PPI/TM | | | | | | *15/70 | |
| EPTC | 6.00 | PPI/TM | 5 | 0.025 | PPI/TM | | | | | | 90/90 | |
| EPTC | 6.00 | PPI/TM | 5 | 0.012 | PPI/TM | | | | | | 90/90 | |
| VERNAM | 1.25 | PPI | 6 | 5.00 | IF | *70/100 | 95/95 | 60/60 | *85/97 | 90/90 | | |
| VERNAM | 6.00 | PPI | 6 | 5.00 | IF | | | | | | *25/95 | 65/65 |
| EPTC | 6.00 | PPI/TM | 6 | 0.05 | PPI/TM | | | | | | *35/80** | |
| EPTC | 6.00 | PPI/TM | 6 | 0.50 | PPI/TM | | | | | | *20/70 | |
| EPTC | 6.00 | PPI/TM | 6 | 5.00 | PPI/TM | | | | | | *15/70 | |
| EPTC | 6.00 | PPI/TM | 6 | 0.025 | PPI/TM | | | | | | *65/90 | |
| EPTC | 6.00 | PPI/TM | 6 | 0.012 | PPI/TM | | | | | | 80/90 | |

TABLE III
(continued)

| Herbicide | | | Antidote | | | % Injury | | | | | | |
|---|---|---|---|---|---|---|---|---|---|---|---|---|
| Name | Rate lb/A | Method | Cmpd. No. | Rate lb/A | Method | Milo | Wheat | Cotton | Rice | Barley | Corn | Soybean |
| VERNAM | 1.25 | PPI | 7 | 5.00 | IF | *80/100 | 95/95 | 50/60 | 97/97 | 90/90 | | |
| VERNAM | 6.00 | PPI | 7 | 5.00 | IF | | | | | | *15/95 | 75/65 |
| EPTC | 6.00 | PPI/TM | 7 | 0.05 | PPI/TM | | | | | | *53/80** | |
| EPTC | 6.00 | PPI/TM | 7 | 0.50 | PPI/TM | | | | | | *10/70 | |
| EPTC | 6.00 | PPI/TM | 7 | 5.00 | PPI/TM | | | | | | * 0/70 | |
| EPTC | 6.00 | PPI/TM | 7 | 0.025 | PPI/TM | | | | | | 90/90 | |
| EPTC | 6.00 | PPI/TM | 7 | 0.012 | PPI/TM | | | | | | 90/90 | |
| VERNAM | 1.25 | PPI | 8 | 5.00 | IF | 100/100 | 95/95 | 60/60 | 97/97 | 90/90 | | |
| VERNAM | 6.00 | PPI | 8 | 5.00 | IF | | | | | | *80/95 | 55/65 |
| VERNAM | 1.25 | PPI | 9 | 5.00 | IF | *65/100 | 95/95 | 60/60 | 97/97 | 80/90 | | |
| VERNAM | 6.00 | PPI | 9 | 5.00 | IF | | | | | | *15/95 | 80/65 |
| EPTC | 6.00 | PPI/TM | 9 | 0.05 | PPI/TM | | | | | | *48/80** | |
| EPTC | 6.00 | PPI/TM | 9 | 0.50 | PPI/TM | | | | | | *15/70 | |
| EPTC | 6.00 | PPI/TM | 9 | 5.00 | PPI/TM | | | | | | *20/70 | |
| EPTC | 6.00 | PPI/TM | 9 | 0.025 | PPI/TM | | | | | | *75/90 | |
| EPTC | 6.00 | PPI/TM | 9 | 0.012 | PPI/TM | | | | | | 90/90 | |
| VERNAM | 6.00 | PPI/TM | 9 | 1.00 | PPI/TM | | | | | | | 65/30 |
| VERNAM | 6.00 | PPI/TM | 9 | 2.00 | PPI/TM | | | | | | | 65/30 |
| VERNAM | 6.00 | PPI/TM | 9 | 5.00 | PPI/TM | | | | | | | 80/30 |

TABLE III
(continued)

| Herbicide | | | Antidote | | | % Injury | | | | | | |
|---|---|---|---|---|---|---|---|---|---|---|---|---|
| Name | Rate lb/A | Method | Cmpd. No. | Rate lb/A | Method | Milo | Wheat | Cotton | Rice | Barley | Corn | Soybean |
| VERNAM | 1.25 | PPI | 10 | 5.00 | IF | *75/100 | *75/95 | 60/60 | 97/97 | 90/90 | | |
| VERNAM | 6.00 | PPI | 10 | 5.00 | IF | | | | | | *15/95 | 80/65 |
| EPTC | 6.00 | PPI/TM | 10 | 0.50 | PPI/TM | | | | | | *20/70 | |
| EPTC | 6.00 | PPI/TM | 10 | 5.00 | PPI/TM | | | | | | * 0/70 | |
| EPTC | 6.00 | PPI/TM | 10 | 0.05 | PPI/TM | | | | | | *58/80** | |
| EPTC | 6.00 | PPI/TM | 10 | 0.025 | PPI/TM | | | | | | 80/90 | |
| EPTC | 6.00 | PPI/TM | 10 | 0.012 | PPI/TM | | | | | | 90/90 | |
| VERNAM | 1.25 | PPI | 11 | 5.00 | IF | *75/100 | 85/95 | 60/60 | *85/97 | 85/90 | | |
| VERNAM | 6.00 | PPI | 11 | 5.00 | IF | | | | | | *45/95 | 75/65 |
| VERNAM | 1.25 | PPI | 12 | 5.00 | IF | *75/100 | 95/95 | 65/65 | 98/98 | *75/95 | | |
| VERNAM | 6.00 | PPI | 12 | 5.00 | IF | | | | | | *35/95 | 65/65 |
| EPTC | 6.00 | PPI/TM | 12 | 0.50 | PPI/TM | | | | | | *20/70 | |
| EPTC | 6.00 | PPI/TM | 12 | 5.00 | PPI/TM | | | | | | * 0/70 | |
| EPTC | 6.00 | PPI/TM | 12 | 0.05 | PPI/TM | | | | | | *63/80** | |
| EPTC | 6.00 | PPI/TM | 12 | 0.025 | PPI/TM | | | | | | 90/90 | |
| EPTC | 6.00 | PPI/TM | 12 | 0.012 | PPI/TM | | | | | | 90/90 | |
| VERNAM | 1.25 | PPI | 13 | 5.00 | IF | *70/100 | 85/95 | 65/65 | 98/98 | 85/85 | | |
| VERNAM | 6.00 | PPI | 13 | 5.00 | IF | | | | | | 85/95 | 65/65 |
| VERNAM | 1.25 | PPI | 14 | 5.00 | IF | 100/100 | 95/95 | 65/65 | *60/98 | 75/85 | | |
| VERNAM | 6.00 | PPI | 14 | 5.00 | IF | | | | | | *55/95 | 65/65 |

TABLE III
(continued)

| Herbicide | | | Antidote | | | % Injury | | | | | | |
|---|---|---|---|---|---|---|---|---|---|---|---|---|
| Name | Rate lb/A | Method | Cmpd. No. | Rate lb/A | Method | Milo | Wheat | Cotton | Rice | Barley | Corn | Soybean |
| VERNAM | 1.25 | PPI | 15 | 5.00 | IF | 100/100 | 95/95 | 65/65 | 98/98 | 85/85 | | |
| VERNAM | 6.00 | PPI | 15 | 5.00 | IF | | | | | | 90/95 | *40/65 |
| VERNAM | 1.25 | PPI | 16 | 5.00 | IF | 100/100 | 95/95 | 65/65 | 98/98 | 75/85 | | |
| VERNAM | 6.00 | PPI | 16 | 5.00 | IF | | | | | | 95/95 | 65/65 |
| VERNAM | 1.25 | PPI | 18 | 5.00 | IF | 100/100 | 95/95 | 50/50 | 100/100 | 95/95 | | |
| VERNAM | 6.00 | PPI | 18 | 5.00 | IF | | | | | | *70/90 | 60/60 |
| VERNAM | 1.25 | PPI | 19 | 5.00 | IF | *65/100 | 85/95 | 40/50 | 100/100 | 95/95 | | |
| VERNAM | 6.00 | PPI | 19 | 5.00 | IF | | | | | | *20/90 | 70/60 |
| EPTC | 6.00 | PPI/TM | 19 | 0.50 | PPI/TM | | | | | | * 0/75 | |
| EPTC | 6.00 | PPI/TM | 19 | 5.00 | PPI/TM | | | | | | *10/75 | |
| EPTC | 6.00 | PPI/TM | 19 | 0.05 | PPI/TM | | | | | | * 8/78** | |
| EPTC | 6.00 | PPI/TM | 19 | 0.025 | PPI/TM | | | | | | *35/80 | |
| EPTC | 6.00 | PPI/TM | 19 | 0.012 | PPI/TM | | | | | | *65/80 | |
| VERNAM | 1.25 | PPI | 20 | 5.00 | IF | 100/100 | 95/95 | 50/50 | 100/100 | 95/95 | | |
| VERNAM | 6.00 | PPI | 20 | 5.00 | IF | | | | | | *75/90 | 60/60 |

TABLE IV

Herbicidal Effectiveness

| Herbicide | | | Antidote | | | % Injury | | | |
|---|---|---|---|---|---|---|---|---|---|
| Name | Rate lb/A | Method | Cmpd. No. | Rate lb/A | Method | Water-grass | Foxtail | Wild Oat | Shatter-cane |
| RONEET | 3.00 | PPI | 1 | 5.00 | IF | | 100/95 | | 100/98 |
| RONEET | 3.00 | PPI | 1 | 5.00 | IF | | 95/95 | | 100/98 |
| RONEET | 3.00 | PPI/IM | 1 | 1.00 | PPI/IM | | 95/95 | | 95/95 |
| RONEET | 3.00 | PPI/IM | 1 | 2.00 | PPI/IM | | 95/95 | | 95/95 |
| RONEET | 3.00 | PPI/IM | 1 | 5.00 | PPI/IM | | 75/95 | | 100/95 |
| RONEET | 3.00 | PPI/IM | 1 | 5.00 | PPI/IM | 90/90 | 75/75 | | |
| RONEET | 3.00 | PPI/IM | 1 | 1.00 | PPI/IM | 90/90 | 75/75 | | |
| RONEET | 3.00 | PPI/IM | 1 | 0.50 | PPI/IM | 90/90 | 75/75 | | |
| RONEET | 3.00 | PPI/IM | 1 | 0.25 | PPI/IM | 90/90 | 75/75 | | |
| EPTC | 6.00 | PPI/IM | 1 | 0.05 | PPI/IM | 100/100 | 100/100 | | |
| EPTC | 6.00 | PPI/IM | 1 | 0.50 | PPI/IM | 100/100 | 100/100 | | |
| EPTC | 6.00 | PPI/IM | 1 | 5.00 | PPI/IM | 100/100 | 100/100 | | |
| EPTC | 6.00 | PPI/IM | 1 | 0.025 | PPI/IM | 100/100 | 100/100 | | |
| EPTC | 6.00 | PPI/IM | 1 | 0.012 | PPI/IM | 100/100 | 100/100 | | |
| LASSO | 3.50 | PES | 1 | 5.00 | IF | 100/100 | | | |
| TERIDOX | 1.00 | PES | 1 | 5.00 | IF | 100/100 | | | |
| SUTAN | 6.00 | PPI | 1 | 1.00 | IF | 100/100 | | | 100/100 |
| SUTAN | 6.00 | PPI | 1 | 5.00 | IF | 100/100 | | | 100/100 |
| VERNAM | 6.00 | PPI/IM | 1 | 1.00 | PPI/IM | 100/100 | 95/95 | | |
| VERNAM | 6.00 | PPI/IM | 1 | 2.00 | PPI/IM | 100/100 | 95/95 | | |
| VERNAM | 6.00 | PPI/IM | 1 | 5.00 | PPI/IM | 100/100 | 95/95 | | |
| VERNAM | 5.00 | PPI | 1 | 1.00 | IF | 95/95 | | | 100/100 |
| VERNAM | 5.00 | PPI | 1 | 5.00 | IF | 95/95 | | | 100/100 |
| RONEET | 3.00 | PPI | 3 | 5.00 | IF | | 95/95 | | 100/100 |
| RONEET | 3.00 | PPI | 3 | 1.00 | IF | | 95/95 | | 100/100 |
| RONEET | 3.00 | PPI/IM | 3 | 1.00 | PPI/IM | | 90/90 | | 100/100 |
| RONEET | 3.00 | PPI/IM | 3 | 2.00 | PPI/IM | | 90/90 | | 100/100 |
| RONEET | 3.00 | PPI/IM | 3 | 5.00 | PPI/IM | | 90/90 | | 100/100 |
| RONEET | 3.00 | PPI | 4 | 1.00 | IF | | 95/95 | | 100/98 |
| RONEET | 3.00 | PPI | 4 | 5.00 | IF | | 95/95 | | 98/98 |
| EPTC | 6.00 | PPI/IM | 4 | 0.05 | PPI/IM | 90/90 | 95/95 | | |
| EPTC | 6.00 | PPI/IM | 4 | 0.50 | PPI/IM | 90/90 | 95/95 | | |
| EPTC | 6.00 | PPI/IM | 4 | 5.00 | PPI/IM | 90/90 | 95/95 | | |
| EPTC | 6.00 | PPI/IM | 4 | 0.025 | PPI/IM | 100/100 | 100/100 | | |
| EPTC | 6.00 | PPI/IM | 4 | 0.012 | PPI/IM | 100/100 | 100/100 | | |

TABLE IV

(Continued)

| Herbicide | | | Antidote | | | % Injury | | | |
|---|---|---|---|---|---|---|---|---|---|
| Name | Rate lb/A | Method | Cmpd. No. | Rate lb/A | Method | Water-grass | Foxtail | Wild Oat | Shatter-cane |
| RONEET | 3.00 | PPI/IM | 4 | 1.00 | PPI/IM | | 95/95 | | 95/95 |
| RONEET | 3.00 | PPI/IM | 4 | 2.00 | PPI/IM | | 100/95 | | 100/95 |
| RONEET | 3.00 | PPI/IM | 4 | 5.00 | PPI/IM | | 95/95 | | 95/95 |
| RONEET | 3.00 | PPI | 4 | 1.00 | IF | | 95/95 | | 100/100 |
| RONEET | 3.00 | PPI | 4 | 5.00 | IF | | 70/95 | | 100/100 |
| RONEET | 3.00 | PPI/IM | 4 | 5.00 | PPI/IM | 90/90 | 75/75 | | |
| RONEET | 3.00 | PPI/IM | 4 | 1.00 | PPI/IM | 90/90 | 75/75 | | |
| RONEET | 3.00 | PPI/IM | 4 | 0.50 | PPI/IM | 90/90 | 75/75 | | |
| RONEET | 3.00 | PPI/IM | 4 | 0.25 | PPI/IM | 90/90 | 75/75 | | |
| RONEET | 3.00 | PPI/IM | 4 | 2.00 | PPI/IM | 100/100 | | | 80/100 |
| RONEET | 3.00 | PPI/IM | 4 | 2.00 | PPI/IM | 100/100 | | | 80/100 |
| RONEET | 3.00 | PPI/IM | 4 | 0.50 | PPI/IM | 100/100 | | | 80/100 |
| RONEET | 3.00 | PPI/IM | 4 | 0.25 | PPI/IM | 100/100 | | | 85/100 |
| RONEET | 3.00 | PPI/IM | 4 | 0.125 | PPI/IM | 100/100 | | | 85/100 |
| RONEET | 3.00 | PPI/IM | 4 | 0.06 | PPI/IM | 100/100 | | | 100/100 |
| VERNAM | 5.00 | PPI/IM | 4 | 1.00 | PPI/IM | 95/95 | 100/100 | | |
| VERNAM | 5.00 | PPI/IM | 4 | 2.00 | PPI/IM | 95/95 | 100/100 | | |
| VERNAM | 5.00 | PPI/IM | 4 | 5.00 | PPI/IM | 95/95 | 100/100 | | |
| EPTC | 6.00 | PPI/IM | 5 | 0.05 | PPI/IM | 90/90 | 95/95 | | |
| EPTC | 6.00 | PPI/IM | 5 | 0.50 | PPI/IM | 95/95** | 98/98** | | |
| EPTC | 6.00 | PPI/IM | 5 | 5.00 | PPI/IM | 90/90 | 95/95 | | |
| EPTC | 6.00 | PPI/IM | 5 | 0.025 | PPI/IM | 100/100 | 100/100 | | |
| EPTC | 6.00 | PPI/IM | 5 | 0.012 | PPI/IM | 100/100 | 100/100 | | |
| EPTC | 6.00 | PPI/IM | 6 | 0.05 | PPI/IM | 95/95** | 98/98** | | |
| EPTC | 6.00 | PPI/IM | 6 | 0.50 | PPI/IM | 90/90 | 95/95 | | |
| EPTC | 6.00 | PPI/IM | 6 | 5.00 | PPI/IM | 90/90 | 95/95 | | |
| EPTC | 6.00 | PPI/IM | 6 | 0.025 | PPI/IM | 100/100 | 100/100 | | |
| EPTC | 6.00 | PPI/IM | 6 | 0.012 | PPI/IM | 100/100 | 100/100 | | |
| EPTC | 6.00 | PPI/IM | 7 | 0.05 | PPI/IM | 95/95** | 98/98** | | |
| EPTC | 6.00 | PPI/IM | 7 | 0.50 | PPI/IM | 90/90 | 95/95 | | |
| EPTC | 6.00 | PPI/IM | 7 | 5.00 | PPI/IM | 90/90 | 95/95 | | |
| EPTC | 6.00 | PPI/IM | 7 | 0.025 | PPI/IM | 100/100 | 100/100 | | |
| EPTC | 6.00 | PPI/IM | 7 | 0.012 | PPI/IM | 100/100 | 100/100 | | |

TABLE IV

(Continued)

| Herbicide | | | Antidote | | | % Injury | | | |
|---|---|---|---|---|---|---|---|---|---|
| Name | Rate lb/A | Method | Cmpd. No. | Rate lb/A | Method | Water-grass | Foxtail | Wild Oat | Shatter-cane |
| EPTC | 6.00 | PPI/IM | 9 | 0.05 | PPI/IM | 95/95** | 98/98** | | |
| EPTC | 6.00 | PPI/IM | 9 | 0.50 | PPI/IM | 90/90 | 95/95 | | |
| EPTC | 6.00 | PPI/IM | 9 | 5.00 | PPI/IM | 90/90 | 95/95 | | |
| EPTC | 6.00 | PPI/IM | 9 | 0.025 | PPI/IM | 95/95** | 98/98** | | |
| EPTC | 6.00 | PPI/IM | 9 | 0.012 | PPI/IM | 100/100 | 100/100 | | |
| VERNAM | 6.00 | PPI/IM | 9 | 1.00 | PPI/IM | 95/95 | 95/95 | | |
| VERNAM | 6.00 | PPI/IM | 9 | 2.00 | PPI/IM | 95/95 | 95/95 | | |
| VERNAM | 6.00 | PPI/IM | 9 | 5.00 | PPI/IM | 95/95 | 95/95 | | |
| EPTC | 6.00 | PPI/IM | 10 | 0.05 | PPI/IM | 95/95** | 98/98** | | |
| EPTC | 6.00 | PPI/IM | 10 | 0.50 | PPI/IM | 90/90 | 95/95 | | |
| EPTC | 6.00 | PPI/IM | 10 | 5.00 | PPI/IM | 90/90 | 95/95 | | |
| EPTC | 6.00 | PPI/IM | 10 | 0.025 | PPI/IM | 100/100 | 100/100 | | |
| EPTC | 6.00 | PPI/IM | 10 | 0.012 | PPI/IM | 100/100 | 100/100 | | |
| EPTC | 6.00 | PPI/IM | 12 | 0.05 | PPI/IM | 95/95** | 98/98** | | |
| EPTC | 6.00 | PPI/IM | 12 | 0.50 | PPI/IM | 90/90 | 95/95 | | |
| EPTC | 6.00 | PPI/IM | 12 | 5.00 | PPI/IM | 90/90 | 95/95 | | |
| EPTC | 6.00 | PPI/IM | 12 | 0.025 | PPI/IM | 100/100 | 100/100 | | |
| EPTC | 6.00 | PPI/IM | 12 | 0.012 | PPI/IM | 100/100 | 100/100 | | |
| EPTC | 6.00 | PPI/IM | 19 | 0.05 | PPI/IM | 95/95** | 100/100** | | |
| EPTC | 6.00 | PPI/IM | 19 | 0.50 | PPI/IM | 100/100 | 100/100 | | |
| EPTC | 6.00 | PPI/IM | 19 | 5.00 | PPI/IM | 100/100 | 100/100 | | |
| EPTC | 6.00 | PPI/IM | 19 | 0.05 | PPI/IM | 90/90 | 100/100 | | |
| EPTC | 6.00 | PPI/IM | 19 | 0.025 | PPI/IM | 90/90 | 100/100 | | |

TEST RESULTS

The compounds of this invention show good antidotal activity with a variety of crops, especially with milo and corn. Use of the antidote compounds, as indicated in Table IV, did not result in a reduction of herbicidal injury to weeds.

Formulations

A formulation is the incorporation of a formulant in a form which is directly usable on crops and weeds. As defined herein, a "formulant" is the material which is to be formulated. The formulant may be

either an antidote compound alone or an herbicide and antidote composition. The purpose of the formulation is to apply the formulant to the locus where it is desired to establish herbicidal selectivity by a convenient method. The "locus" may include soil, seeds, seedlings and vegetation.

The formulations are commonly dusts, wettable powders, granules, solutions or emulsifiable concentrates.

Dusts are free-flowing powder compositions containing the formulant impregnated on a particulate carrier. The particle size of the carriers is usually in the approximate range of 30 to 50 microns. Examples of suitable carriers are talc, bentonite, diatomaceous earth, and pyrophyllite. The composition generally contains up to 50% of formulant. Anti-caking and anti-static agents may also be added. Dusts may be applied by spraying from boom and hand sprayers on airplanes.

Wettable powders are finely divided compositions comprising a particulate carrier impregnated with the formulant and additionally containing one or more surface active agents. The surface active agent promotes rapid dispersion of the powder in an aqueous medium to form stable, sprayable suspensions. A wide variety of surface active agents can be used, for example, long chain fatty alcohols and alkali metal salts of the sulfated fatty alcohols; salts of sulfonic acid; esters of long chain fatty acids; and polyhydric alcohols, in which the alcohol groups are free, omega-substituted polyethylene glycols of relatively long chain length. A list of surface active agents suitable for use in agriculture formulations can be found in Wade Van Valkenburg, Pesticide Formulations (Marcel Dekker, Inc., N.Y., 1973) at pages 79-84.

Granules comprise the formulant impregnated on a particulate inert carrier having a particle size of about 1 to 2 millimeters (mm) in diameter. The granules can be made by spraying a solution of the formulant in a volatile solvent onto the granular carrier. Examples of suitable carriers for the preparation of granules include clay, vermiculite, sawdust, and granular carbon.

Emulsifiable concentrates consist of an oil solution of the formulant plus an emulsifying agent. Prior to use the concentrate is diluted with water to form a suspended emulsion of oil droplets. The emulsifiers used are usually a mixture of anionic and nonionic surfactants. Other additives, such as suspending agents and thickeners, may be included in the emulsifiable concentrate.

When the formulant is an antidote and herbicide composition, the proportion of antidote compound to herbicide compound generally ranges from approximately 0.001 to 30 parts by weight of the antidote compound per weight of the herbicide compound.

Formulations generally contain several additives in addition to the formulant and carrier or agent. Among these are inert ingredients, diluent carriers, organic solvents, water, oil and water, water in oil emulsions, carriers of dusts and granules, and surface active wetting, dispersing and emulsifying agents. Fertilizers, e.g., ammonium nitrate, urea and superphosphate, may be included. Aids to rooting and growth, e.g., compost, manure, humus and sand, may also be included.

Alternatively, the antidote compounds and herbicide and antidote compositions of this invention can be applied to a crop by addition of the formulant to irrigation water supplied to the field to be treated. This method of application permits the penetration of the compositions into the soil as the water is absorbed.

As another alternative, the formulant can be applied to the soil in the form of a solution in a suitable solvent. Solvents frequently used in these formulations include kerosene, fuel oil, xylene, petroleum fractions with boiling ranges above xylene and aromatic petroleum fractions rich in methylated naphthalenes. Liquid solutions, like dusts, may be applied by spraying from boom and hand sprayers on airplanes.

CLAIMS:

1. A compound according to the formula

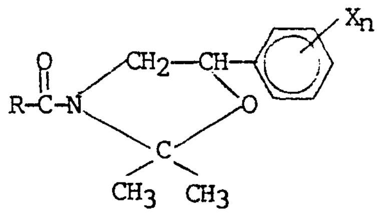

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_2$-$C_4$ haloalkyl or halogen; and

n is one of the integers 1 to 5, inclusive,

with the proviso that where X is trifluoromethyl, R is other than 3-chloropropyl.

2. A compound according to Claim 1 wherein R is $C_1$-$C_4$ haloalkyl, $C_1$-$C_4$ alkyl, $C_1$-$C_4$ cycloalkyl, $C_1$-$C_4$ haloalkenyl, $C_1$-$C_4$ alkylthio; X is $C_1$-$C_2$ alkyl, $C_1$-$C_2$ alkoxy, $C_1$-$C_2$ haloalkyl, chlorine, bromine or fluorine; and n is one of the integers 1, 2 or 3.

3. A compound according to Claim 1 wherein R is pentadecanyl, cyclopropyl, propylthio or methyl, ethyl, propyl, or vinyl groups which are mono-, di-, or tri-substituted with chlorine or bromine; and X is methyl, methoxy, halogen or trifluoromethyl.

4. A compound according to Claim 3 wherein R is monochloro- or dichloromethyl; X is para- or meta-methoxy, meta-trifluoromethyl, para-methyl or para-chloro; and n is one of the integers 1 or 2.

5. A compound according to Claim 4 wherein R is dichloromethyl, X is para-methoxy or meta-trifluoromethyl; and n is the integer 1.

6. An herbicidal composition comprising:

(a) an herbicidally effective amount of a thiocarbamate of the formula

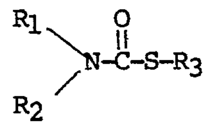

in which

$R_1$ is alkyl having 1-6 carbon atoms, inclusive;

$R_2$ is selected from the group consisting of alkyl having 1-6 carbon atoms, inclusive; and cyclohexyl; or

$R_1$ and $R_2$ form indistinguishable parts of a single alkylene ring having 4-10 carbon atoms, inclusive; and

$R_3$ is selected from the group consisting of alkyl having 1-6 carbon atoms, inclusive; haloalkyl wherein halo is selected from the group consisting of chlorine, bromine and iodine; alkenyl having 2-6 carbon atoms, inclusive; haloalkenyl wherein halo is selected from the group consisting of chlorine, bromine an iodine and alkenyl has 2-6 carbon atoms, inclusive; benzyl and halo-substituted benzyl, wherein halo is selected from the group consisting of chlorine, bromine and iodine; and

(b) a non-phytotoxic antidotally effective amount of a compound of the formula

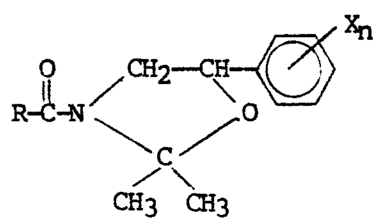

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

7. An herbicidal composition according to Claim 6 wherein $R_1$ and $R_2$ are independently each $C_1$-$C_6$ alkyl; and $R_2$ is either $C_1$-$C_6$ alkyl or $C_1$-$C_8$ cycloalkyl.

8. An herbicidal composition according to Claim 7 wherein R is $C_1$-$C_4$ haloalkyl, $C_1$-$C_4$ alkyl, $C_1$-$C_4$ cycloalkyl, $C_1$-$C_4$ haloalkenyl, $C_1$-$C_4$ alkylthio; X is $C_1$-$C_2$ alkyl, $C_1$-$C_2$ alkoxy, $C_1$-$C_2$ haloalkyl, chlorine, bromine or fluorine; and n is one of the integers 1, 2 or 3.

9. An herbicidal composition according to Claim 8 wherein R is pentadecanyl, cyclopropyl, propylthio or methyl, ethyl, propyl, or vinyl groups which are mono-, di-, or tri-substituted with chlorine or bromine; and X is methyl, methoxy, halogen or trifluoromethyl.

10. An herbicidal composition according to Claim 9 wherein R is monochloro- or dichloromethane; and X is para-chloro, para-methyl, para-methoxy, meta-trifluoromethyl, or meta-methoxy.

11. An herbicidal composition according to Claim 10 wherein said thiocarbamate herbicides are S-ethyl N,N-dipropyl thiocarbamate; S-ethylN-ethyl-N-cyclohexyl thiocarbamate; S-propyl N,N-dipropyl thiocarbamate; or S-ethyl diisobutyl thiocarbamate.

12. An herbicidal composition according to Claim 10 wherein R is dichloromethyl; X is para-methoxy or meta-trifluoromethyl; and n is the integer 1.

13. An herbicidal composition according to Claim 12 wherein the thiocarbamate herbicide is either S-propyl N,N-dipropyl thiocarbamate or S-ethyl-N-ethyl-N-cyclohexyl thiocarbamate and the antidote is either 2,2-dimethyl-3-(dichloroacetyl)-5-(p-methoxyphenyl)-1,3-oxazolidine or 2,2-dimethyl-3-dichloroacetyl-5-(m-trifluoromethylphenyl)-1,3-oxazolidine.

14. An herbicidal composition according to Claim 13 wherein the thiocarbamate herbicide is S-ethyl-N-ethyl-N-cyclohexyl thiocarbamate and the antidote is 2,2-dimethyl-3-dichloroacetyl-5-(m-trifluoromethylphenyl)-1,3-oxazolidine.

15. An herbicidal composition comprising:

(a) an herbicidally effective amount of an acetanilide compound of the formula

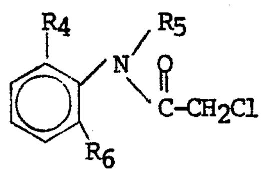

in which

$R_4$ and $R_6$ are each independently alkyl having 1-4 carbon atoms, inclusive; and

$R^5$ is alkoxyalkyl having 1-4 carbon atoms; and

(b) a non-phytotoxic antidotally effective amount of a compound of the formula

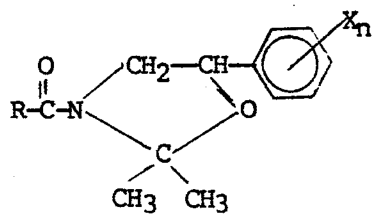

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

16. An herbicidal composition according to Claim 15 wherein R is $C_1$-$C_4$ haloalkyl, $C_1$-$C_4$ alkyl, $C_1$-$C_4$ cycloalkyl, $C_1$-$C_4$ haloalkenyl, $C_1$-$C_4$ alkylthio; X is $C_1$-$C_2$ alkyl, $C_1$-$C_2$ alkoxy, $C_1$-$C_2$ haloalkyl, chlorine, bromine or fluorine; and n is one of the integers 1, 2 or 3.

17. An herbicidal composition according to Claim 156wherein R is pentadecanyl, cyclopropyl, propylthio or methyl, ethyl, propyl, or vinyl groups which are mono-, di-, or tri-substituted with chlorine or bromine; X is methyl, methoxy, chlorine or trifluoromethyl.

18. An herbicidal composition according to Claim 17 wherein R is dichloromethyl; X is para-methoxy; and n is the integer 1.

19. An herbicidal composition according to Claim 15 wherein the acetanilide herbicide is either 2-chloro-2',6'-dimethyl-N-(methoxyethyl) acetanilide or 2-chloro-2',6'-diethyl-N-(methoxymethyl) acetanilide.

20. A method of controlling undesirable vegetation and reducing herbicidal crop injury caused by acetanilide or thiocarbamate herbicides which comprises applying to the locus where control is desired a compound of the formula

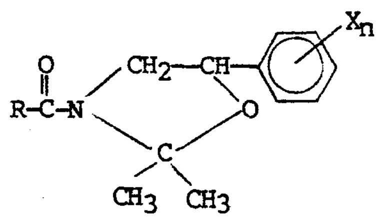

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is one of the integers 1 to 5, inclusive,

with the proviso that where X is trifluoromethyl, R is other than 3-chloropropyl.

21. A method of controlling undesirable vegetation and reducing herbicidal crop injury due to a thiocarbamate herbicide which comprises applying to the locus where control is desired a herbicidal composition comprising:

(a) an herbicidally effective amount of a thiocarbamate of the formula

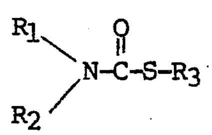

in which

$R_1$ and $R_3$ are each independently $C_1$-$C_6$ alkyl; and

$R_2$ is either $C_1$-$C_6$ alkyl or $C_1$-$C_8$ cycloalkyl; and

(b) a non-phytotoxic antidotally effective amount of a compound of the formula

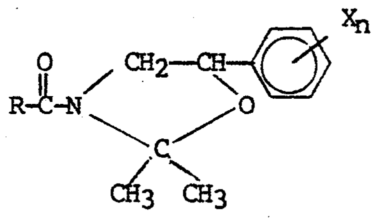

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

CLAIMS:

1. A method of controlling undesirable vegetation and reducing herbicidal crop injury caused by acetanilide or thiocarbamate herbicides which comprises applying to the locus where control is desired a compound of the formula:

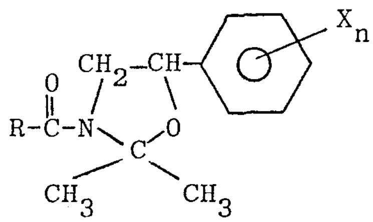

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is one of the integers 1 to 5, inclusive,

with the proviso that where X is trifluoromethyl, R is other than 3-chloropropyl.

2. A method of controlling undesirable vegetation and reducing herbicidal crop injury due to a thiocarbamate herbicide which comprises applying to the locus where control is desired a herbicidal composition comprising:

(a) an herbicidally effective amount of a thiocarbamate of the formula:

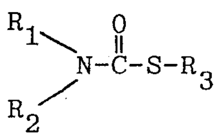

in which

$R_1$ and $R_3$ are each independently $C_1$-$C_6$ alkyl; and

$R_2$ is either $C_1$-$C_6$ alkyl or $C_1$-$C_8$ cycloalkyl; and

(b) a non-phytotoxic antidotally effective amount of a compound of the formula:

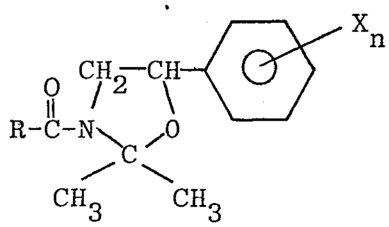

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_4$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

3. An herbicidal composition comprising:

(a) an herbicidally effective amount of a thiocarbamate of the formula:

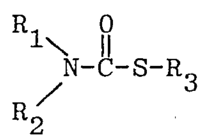

in which

$R_1$ is alkyl having 1-6 carbon atoms, inclusive;

$R_2$ is selected from the group consisting of alkyl having 1-6 carbon atoms, inclusive; and cyclohexyl; or

$R_1$ and $R_2$ form indistinguishable parts of a single alkylene ring having 4-10 carbon atoms, inclusive; and

$R_3$ is selected from the group consisting of alkyl having 1-6 carbon atoms, inclusive; haloalkyl wherein halo is selected from the group consisting of chlorine, bromine and iodine; alkenyl having 2-6 carbon atoms, inclusive; haloalkenyl wherein halo is selected from the group consisting of chlorine, bromine an iodine and alkenyl has 2-6 carbon atoms, inclusive; benzyl and halo-substituted benzyl, wherein halo is selected from the group consisting of chlorine, bromine and iodine; and

(b) a non-phytotoxic antidotally effective amount of a compound of the formula:

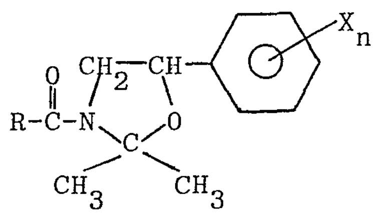

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

4. An herbicidal composition as claimed in claim 3 characterised in that $R_1$ and $R_2$ are independently each $C_1$-$C_6$ alkyl; and $R_2$ is either $C_1$-$C_6$ alkyl or $C_1$-$C_8$ cycloalkyl.

5. An herbicidal composition as claimed in claim 4 characterised in that R is $C_1$-$C_4$ haloalkyl, $C_1$-$C_4$ alkyl, $C_1$-$C_4$ cycloalkyl, $C_1$-$C_4$ haloalkenyl, $C_1$-$C_4$ alkylthio; X is $C_1$-$C_2$ alkyl, $C_1$-$C_2$ alkoxy, $C_1$-$C_2$-haloalkyl, chlorine, bromine or fluorine; and n is one of the integers 1, 2 or 3.

6. An herbicidal composition as claimed in claim 5 characterised in that R is pentadecanyl, cyclopropyl, propylthio or methyl, ethyl, propyl, or vinyl groups which are mono-, di-, or tri-substituted with chlorine or bromine; and X is methyl, methoxy, halogen or trifluoromethyl.

7. An herbicidal composition as claimed in claim 6 characterised in that R is monochloro- or dichloromethane; and X is para-chloro, para-methyl, para-methoxy, meta-trifluoromethyl, or meta-methoxy.

8. An herbicidal composition as claimed in any of claims 3 to 7 characterised in that thiocarbamate herbicides are S-ethyl N,N-dipropyl thiocarbamate; S-ethyl-N-ethyl-N-cyclohexyl thiocarbamate; S-propyl N,N-dipropyl thiocarbamate; or S-ethyl diisobutyl thiocarbamate.

9. An herbicidal composition as claimed in claim 7 characterised in that R is dichloromethyl; X is para-methoxy or meta-trifluoromethyl; and n is the integer 1.

10. An herbicidal composition as claimed in claim 9 characterised in that the thiocarbamate herbicide is either S-propyl N,N-dipropyl thiocarbamate or S-ethyl-N-ethyl-N-cyclohexyl thiocarbamate and the antidote is either 2,2-dimethyl-3-(dichloroacetyl)-5-(p-methoxyphenyl)-1,3-oxazolidine or 2,2-dimethyl-3-dichloroacetyl-5-(m-trifluoromethylphenyl)-1,3-oxazolidine.

11. An herbicidal composition as claimed in claim 10 characterised in that the thiocarbamate herbicide is S-ethyl-N-ethyl-N-cyclohexyl thiocarbamate and the antidote is 2,2-dimethyl-3-dichloroacetyl-5-(m-trifluoromethylphenyl)-1,3-oxazolidine.

12. An herbicidal composition comprising:

(a) an herbicidally effective amount of an acetanilide compound of the formula:

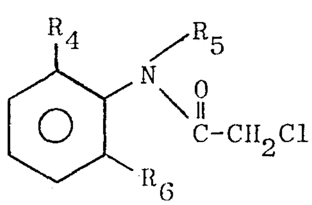

in which

$R_4$ and $R_6$ are each independently alkyl having 1-4 carbon atoms, inclusive; and

$R_5$ is alkoxyalkyl having 1-4 carbon atoms; and

(b) a non-phytotoxic antidotally effective amount of a compound of the formula:

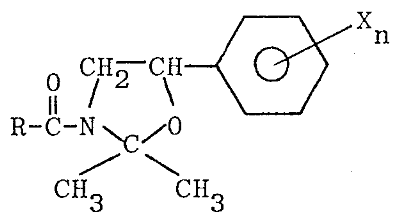

wherein

R is $C_1$-$C_{10}$ haloalkyl, $C_1$-$C_{20}$ alkyl, $C_1$-$C_6$ cycloalkyl, $C_1$-$C_6$ haloalkenyl or $C_1$-$C_6$ alkylthio;

X is $C_1$-$C_4$ alkyl, $C_1$-$C_4$ alkoxy, $C_1$-$C_4$ haloalkyl or halogen; and

n is the integer 1 to 5, inclusive,

with the proviso that where X is trifluoromethyl, R is other than 3-chloropropyl.

13. An herbicidal composition as claimed in claim 12 characterised in that R is $C_1$-$C_4$ haloalkyl, $C_1$-$C_4$ alkyl, $C_1$-$C_4$ cycloalkyl, $C_1$-$C_4$ haloalkenyl, $C_1$-$C_4$ alkylthio; X is $C_1$-$C_2$ alkyl, $C_1$-$C_2$ alkoxy, $C_1$-$C_2$ haloalkyl, chlorine, bromine or fluorine; and n is one of the integers 1, 2 or 3.

14. An herbicidal composition as claimed in claim 13 characterised in that R is pentadecanyl, cyclopropyl, propylthio or methyl, ethyl, propyl, or vinyl groups which are mono-, di-, or tri-substituted with chlorine or bromine; X is methyl, methoxy, chlorine or trifluoromethyl.

15. An herbicidal composition as claimed in claim 14 characterised in that R is dichloromethyl; X is para-methoxy; and n is the integer 1.

16. An herbicidal composition as claimed in any of claims 12 to 15 characterised in that the acetanilide herbicide is either 2-chloro-2',6'-dimethyl-N-(methoxyethyl) acetanilide or 2-chloro-2',6'-diethyl-N-(methoxymethyl) acetanilide.

17. A process for the preparation of the compounds defined in claim 1 characterised in that an appropriate oxazolidine is reacted with an appropriate acid halide in the presence of a hydrogen halide acceptor to produce the desired N-haloacyl-5-(substituted phenyl)oxazolidine of the formula given in claim 1.

18. A composition comprising a compound as claimed in claim 1 in association with an inert carrier or diluent.